(19) Europäisches Patentamt
European Patent Office
Office européen des brevets

(11) Numéro de publication : **0 259 417 B1**

(12) # FASCICULE DE BREVET EUROPÉEN

(45) Date de publication du fascicule du brevet : **03.01.90**

(51) Int. Cl.$^5$ : **C 07 B 37/10**, C 07 D307/93, B 01 J 31/04

(21) Numéro de dépôt : **87901511.3**

(22) Date de dépôt : **05.03.87**

(86) Numéro de dépôt international : **PCT/FR 87/00055**

(87) Numéro de publication internationale : **WO/8705289 (11.09.87 Gazette 87/20)**

(54) **CATALYSEUR DE COUPLAGE BIARYLIQUE AU RUTHENIUM; NOUVEAUX STEGANOLIDES.**

(30) Priorité : **06.03.86 FR 8603152**

(43) Date de publication de la demande : **16.03.88 Bulletin 88/11**

(45) Mention de la délivrance du brevet : **03.01.90 Bulletin 90/01**

(84) Etats contractants désignés : **AT BE CH DE FR GB IT LI LU NL SE**

(56) Documents cités :
**FR-A- 2 460 908**
**Tetrahedron Letters, vol. 24, no. 29, 1983 (Pergamon Press Ltd, Oxford, GB), M. Taafrout et al., "Neoisostegane, a new bisbenzocyclooctadienolactonic lignan from Steganotaenia araliacea HOCHST", pages 2983-2986, voir le document en entier.**
**Tetrahedron Letters, vol. 24, no. 29, 1983 (Pergamon Press Ltd, Oxford, GB), R.P. Hicks et al., "Neoisostegane, a new bisbenzocyclooctadiene lignan lactone from Steganotaenia araliacea HOCHST", pages 2987-2990, voir le document en entier.**
**Journal of Organic Chemistry, vol. 42, no. 4, 1977 (Columbus, Ohio, USA), A. McKillop et al., "Thallium in organic synthesis. 46. Oxidative coupling of aromatic compounds using thallium (III) trifluoroacetate. Synthesis of biaryls", pages 764-765, voir le document en entier.**

(73) Titulaire : **UNIVERSITE DU MAINE (LE MANS)**
**Route de Laval, BP 535**
**F-72017 Le Mans Cedex (FR)**

(72) Inventeur : **ROBIN, Jean-Pierre**
**28, rue Ste Croix**
**F-72000 Le Mans (FR)**
Inventeur : **LANDAIS, Yannick**
**22, rue de l'Etoile**
**F-72000 Le Mans (FR)**

(74) Mandataire : **Ahner, Francis et al**
**CABINET REGIMBEAU 26, avenue Kléber**
**F-75116 Paris (FR)**

**EP 0 259 417 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Chemical Abstracts, vol. 71, no. 5, 4 août 1969 (Columbus, Ohio, USA), N.F. GOL'DSHLEGER et al., "Oxidative coupling of aromatic compounds in systems involving rtthenium complexes", voir pages 288-289, résumé no. 21783p, IZV. AKAD. NAUK SSSR, SER. KHIM. 1969, (3), 675-9.
Tetrahedron Letters, vol. 27, no. 16, 1986 (Pergamon Press Ltd, Oxford, GB), M. Taafrout et al., "Isolement, étude stéréochimique et synthèse biomimétique du stéganolide A, nouveau lignane bisbenzocyclooctadiénolactonique de stéganotaenia araliacea", pages 1781-1784, voir le document en entier.
Tetrahedron Letters, vol. 27, no. 16, 1986 (Pergamon Press Ltd., Oxford, GB), Y. Landais et al., "Le tétrakis (trifluoroacétate) de ruthénium (IV), nouveau catalyseur a température ambiante du couplage biarylique oxydant non phènolique -première synthèse totale biométique du néoisosrégane-", pages 1785-1788, voir le document en entier.

## Description

La présente invention concerne un procédé de couplage biarylique oxydant intramoléculaire de composés précurseurs comportant deux noyaux aromatiques reliés entre eux par une chaîne hydrocarbonée.

L'invention concerne également un nouveau catalyseur organométallique de couplage biarylique oxydant, ainsi que les nouveaux composés à structure biarylique pontée qui ont pu être obtenus par la mise en œuvre du procédé de l'invention.

Un certain nombre d'alcaloïdes biaryliques pontés ont déjà été synthétisés par couplage biarylique oxydant. C'est ainsi que S. M. Kupchan et coll. ont fait appel à l'oxyfluorure de vanadium comme agent de couplage (S. M. Kupchan, K. K. Chakravarti, N. Yokoryama, J. Pharm. Sci., 985 (1963) ; S. M. Kupchan, A. J. Liepa, V. Kamesvaran, R. F. Bryan, J. Am. Chem. Soc., 6861 (1973) et références citées ; S. M. Kupchan, O. P. Dhingra, C.-K. Kim, V. Kamesvaran, J. Org. Chem., 252 (1978) et références citées ; S. M. Kupchan, C. K. Kim, J. Am. Chem. Soc., 5663 (1975) et références citées).

En outre, le tris(trifluoroacétate) de thallium (III) a également déjà été préconisé comme agent de couplage, par exemple par A. Mc Killop et coll. (A. Mc Killop, A. G. Turrell, E. C. Taylor, J. Org. Chem., 765 (1977)) ainsi que par Cambie et coll. (R. C. Cambie, G. R. Clark, P. A. Craw, P. S. Rutledge, P. D. Woodgate, Aust. J. Chem., 1775 (1984)).

Ces techniques antérieures présentent cependant un certain nombre d'inconvénients. Tout d'abord, elles ne permettent pas de conduire, en fin de manipulation à des mélanges réactionnels propres ; il n'est donc pas facile d'en séparer le produit synthétisé qui ne peut donc être obtenu qu'avec des rendements trop faibles. En outre, il faut ajouter que la manipulation des sels de thallium nécessite de très grandes précautions et que leur récupération du milieu réactionnel pose de gros problèmes pratiques.

Ces inconvénients majeurs font qu'il est inenvisageable de transposer de tels procédés de synthèse à l'échelle industrielle. Enfin, il convient d'ajouter que ces procédés de synthèse de la technique antérieure ne pouvaient être mis en œuvre qu'à des températures bien inférieures à O °C, ce qui constitue un inconvénient additionnel évident.

La présente invention s'est précisément proposé d'écarter tous les inconvénients attachés au procédé de synthèse de la technique antérieure connue.

La présente invention se rapporte à un procédé de couplage biarylique oxydant intramoléculaire de composés précurseurs comportant deux noyaux aromatiques, éventuellement polycondensés et pouvant contenir un ou plusieurs hétéroatomes tels que l'oxygène ou l'azote, reliés entre eux par une chaîne hydrocarbonée linéaire ou ramifiée, saturée ou insaturée, et renfermant éventuellement un ou plusieurs hétéroatomes tels que l'oxygène et/ou l'azote, qui est caractérisé en ce que les précurseurs biaryliques sont cyclisés en présence de tétrakis(trifluoroacétate) de ruthénium (IV), en particulier engendré in situ.

L'invention vise également à titre de nouveaux catalyseurs de couplage biarylique oxydant intramoléculaire, le tétrakis(trifluoroacétate) de ruthénium (IV).

Enfin, la présente invention s'étend également à de nouveaux composés biaryliques qui ont pu être synthétisés par la mise en œuvre du procédé de couplage selon l'invention et qui s'avèrent présenter de très intéressantes propriétés anti-virales, en particulier sur le virus herpès. Ces nouveaux composés peuvent donc également entrer dans la composition de préparations thérapeutiques, en particulier des préparations à usage topique.

D'autres caractéristiques et avantages de la présente invention apparaîtront à la lecture de la description détaillée faite ci-après, notamment en référence à des exemples particuliers de préparation donnés à simple titre d'illustration.

Le procédé selon l'invention concerne, de façon très générale, le couplage biarylique oxydant intramoléculaire conduisant à des composés de structure biarylique pontée de natures très diverses. Ce couplage est réalisé sur des précurseurs comportant deux noyaux aromatiques reliés entre eux par une chaîne hydrocarbonée. Chacun des deux noyaux aromatiques, obligatoirement présent dans la structure des précurseurs, peut être soit monocyclique, soit polycyclique ; il peut aussi comporter un ou plusieurs substituants et contenir un ou plusieurs hétéroatomes tels que l'oxygène et/ou l'azote.

La chaîne hydrocarbonée qui relie les deux noyaux aromatiques, est linéaire ou bien ramifiée et peut également renfermer un ou plusieurs hétéroatomes tels que l'oxygène et/ou l'azote.

De façon générale, le couplage selon l'invention peut être représenté schématiquement comme suit :

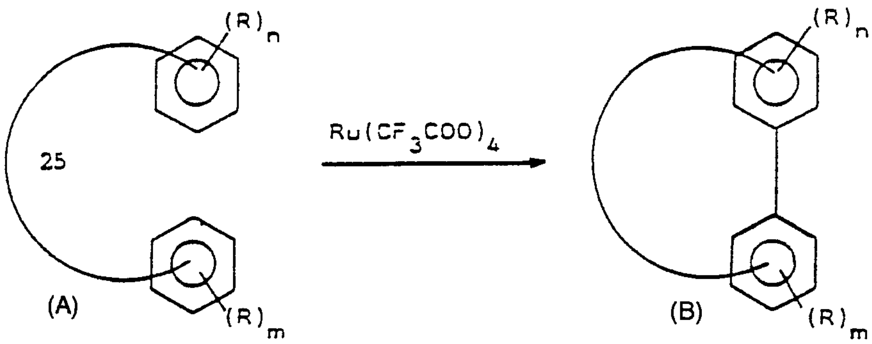

Dans les formules A et B précédentes, les radicaux R désignent des substituants de natures très diverses, en particulier des radicaux alcoyle ou alcoxy(inférieur), par exemple des radicaux méthoxy qui peuvent être présents jusqu'à raison de 3 substituants par cycle. En position para de la position de couplage, les radicaux R peuvent également désigner des groupes hydroxy. Dans ce cas, on observe que le couplage phénolique à l'aide de tétrakis(trifluoroacétate) de ruthénium (IV) s'obtient dans d'excellentes conditions.

D'assez nombreuses classes de substances naturelles de haut intérêt pharmacologique, notamment dans le domaine des anti-tumoraux, présentent la structure biarylique pontée évoquée précédemment. Un certain nombre d'exemples de tels composés ont été mentionnés ci-après à titre de simple illustration. Ils ont été choisis à la fois pour leur représentativité comme chef de file, et pour leur intérêt pharmacologique. Ils peuvent tous être préparés à partir de précurseurs correspondants ouverts, par mise en œuvre du procédé selon l'invention.

| Nom et formule générale de la famille de composés biaryliques pontés | Représentant particulier de la famille de composés biaryliques pontés |
|---|---|
| 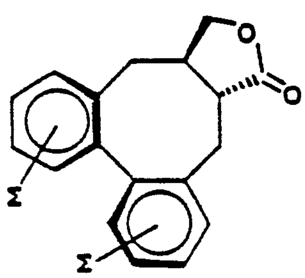<br>C<br>lignanes bisbenzocyclo-octadiènes lactoniques | 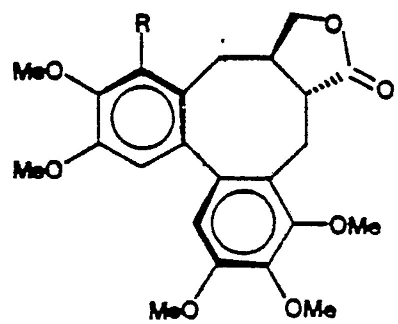<br>1 R = H stéganolide A<br>2 R = OMe néoisostégane |
| 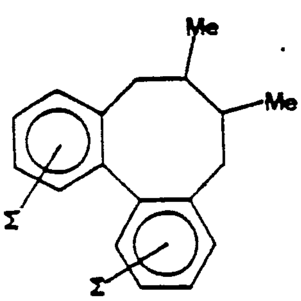<br>D<br>lignanes bisbenzocyclo-octadiènes | 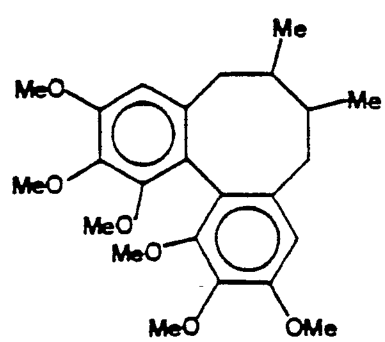<br>3 désoxyschizandrine |

| | |
|---|---|
| 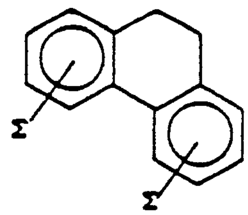<br>E<br>tétrahydrophénantrènes | 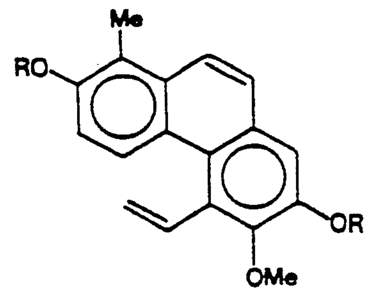<br>4 R = H juncusol |
| 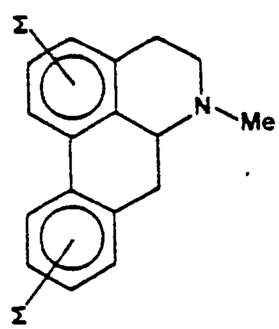<br>F<br>alcaloïdes aporphiniques | 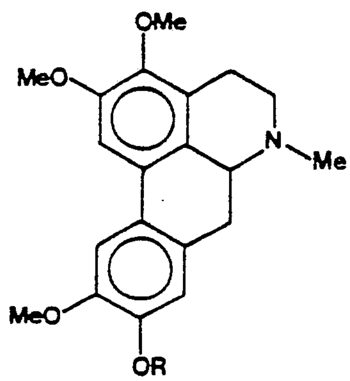<br>5 R = Me glaucine<br>R = 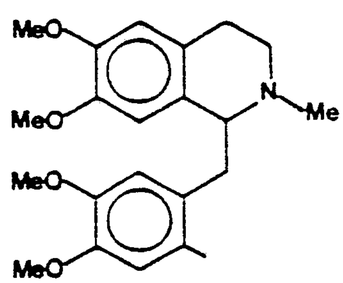<br>6 thalicarpine |

| | |
|---|---|
| 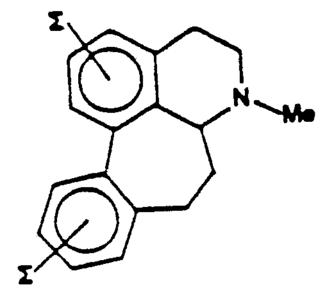<br>G<br>alcaloïdes homoaporphiniques | 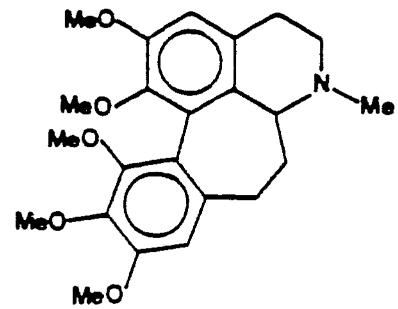<br>7 |
| 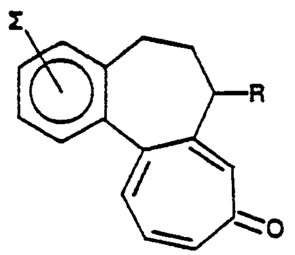<br>H<br>alcaloïdes du groupe de la colchicine | 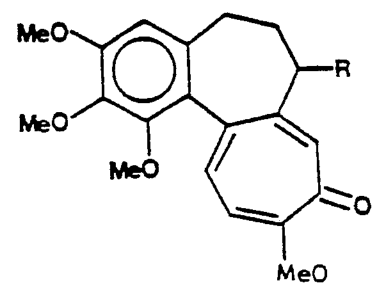<br>8 R = NHAc N-acétylcolchicine |
| 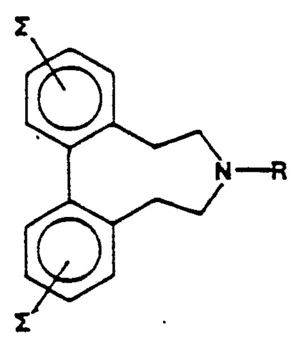<br>I<br>alcaloïdes du groupe des dibenzazonines | 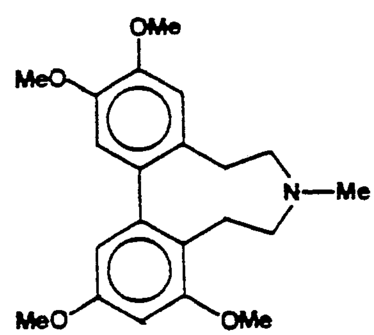<br>9 protostéphanine |

| | |
|---|---|
| 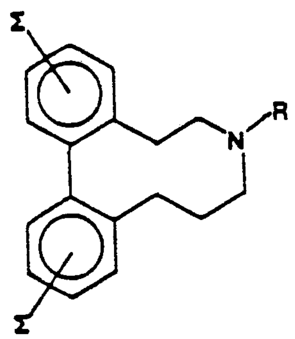<br>J<br>alcaloïdes du groupe des dibenzazécines | 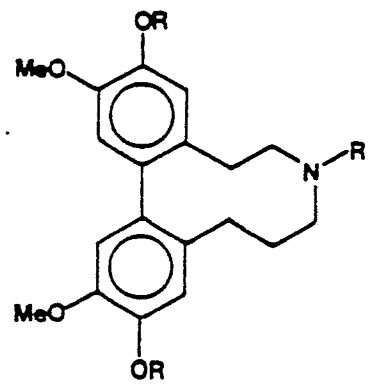<br>10 R = Me |
| 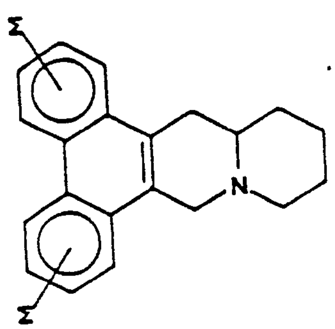<br>K<br>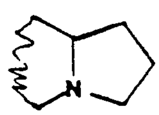<br>L<br>alcaloïdes du groupe des phénanthroquinolizidines (K) et des phénanthroindolizidines (L) | 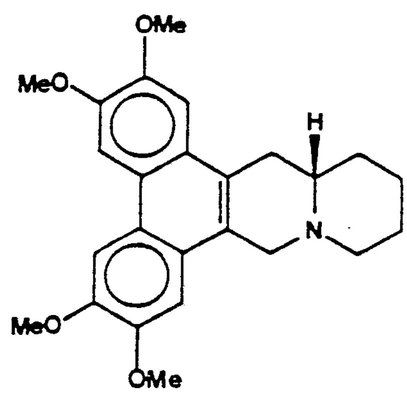<br>11 crytopleurine<br>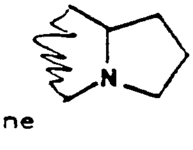<br>12 tylophorine |

On mentionnera ci-après quelques principes généraux de la réaction de couplage selon l'invention, à propos de la synthèse de différents types de dérivés à structure biarylique pontée, mentionnés dans le tableau précédent.

## Synthèse de lignanes bisbenzocyclooctadiènes

Les lignanes 1 à 3 ont été synthétisés à l'aide des précurseurs dibenzyles butanolides correspondants. Dans les exemples présentés, les rendements obtenus pour les composés 1 R = H (néoisostégane), 2 R = OMe (stéganolide A), 3 (désoxyschizandrine) sont quasi quantitatifs (95 à 100 %). Les précurseurs dibenzyles butanolides ont été préparés par une amélioration des procédés de la technique antérieure (E. Brown, J.-P. Robin et R. Dhal, J. C. S. Chem. Comm., 556 (1978) ; M. Taafrout, F. Rouessac et J.-P. Robin, Tetrahedron Letters, 3237 (1983)) à l'aide de la séquence réactionnelle suivante : l'hémiester de Stobbe correspondant a été réduit sélectivement à l'aide du borohydrure de lanthane en milieu éthanolique pour donner, après acidification, les lactones correspondantes. Les dérivés saturés ont été obtenus par hydrogénation catalytiqye (hydrogène, Pd—C), soit au stade hémiester, soit lactone. Ensuite, ces lactones saturées ont été alkylées à l'aide des bromures benzyliques convenablement substitués, en utilisant, soit le diisopropyl amidure de lithium soit, l'hexaméthyldisilyl amidure de lithium comme base dans le tétrahydrofurane, à — 80 °C pour générer l'anion. Cette dernière base s'est avérée particulièrement efficace, puisque certains dibenzyles butanolides ont été obtenus avec un rendement quantitatif. Le précurseur de la désoxyschizandrine 3 a été obtenu par réduction de la lactone (LAH) via le bistosylate du diol primaire correspondant pour donner le dérivé diméthylé, lequel est une lignane ouvert (classe des diarylbutanes). Le couplage biarylique a été ensuite mis en œuvre selon le procédé général décrit plus loin. Le passage aux lignanes présentant la jonction biarylique dite normale suivie de la fonctionnalisation benzylique et de la glycosylation ont été effectués selon la technique antérieure (T. T. Ishiguro, H. Mizuguchi, K. Tomioka, K. Koga, Chem. Pharm. Bull, 33(2) 609 (1985) ; N. Houlbert, E. Brown, J.-P. Robin, J. Nat. Prod., 48, 345 (1985)). Il a été mis en évidence, sur les acétals cycliques correspondants, des propriétés antivirales sur les virus herpès aussi importantes que les produits de référence (Acyclovir). Ces composés peuvent entrer, comme la podophylline, dans la composition de médicaments à usage local (pommade, collyre).

## Synthèse d'alcaloïdes aporphiniques

Le précurseur ouvert des alcaloïdes aporphiniques (formule générale F) de 5 R = Me est disponible commercialement (laudanosine). Il a été soumis à la réaction générale des couplages faisant l'objet de l'invention pour donner la glaucine 5 R = Me avec un rendement de 92 % en composé analytiquement pur. Un certain nombre d'analogues de la thalicarpine 6 ont également été synthétisés.

## Synthèse d'alcaloïdes homoaporphiniques

Les alcaloïdes du groupe homoaporphinique (formule générale G) ont été obtenus par la même méthode, le précurseur benzyléthylisoquinoléique de 7 a été obtenu selon les techniques décrites dans la technique antérieure (S. M. Kupchan, O. P. Dhingra, C. K. Kim, V. Kamesvaran, J. Org. Chem., 252 (1978) et références citées). Le couplage effectué à l'aide du catalyseur au Ru (IV), a donné un rendement de 87 %.

## Méthode générale de couplage biarylique

La méthode générale décrite ci-après s'applique en particulier à tous les composés répondant à la formule générale A. Elle utilise l'oxyde de ruthénium commercial comme précurseur et l'éthérate de trifluorure de bore comme réactif d'assistance électrophile. Les sels organométalliques, qui sont les véritables réactifs, ont été obtenus par attaque de l'oxyde à l'aide de l'acide trifluoroacétique, en présence de l'anhydride trifluoroacétique. Les sels n'ont pas été isolés, toutefois par analogie avec les travaux cités pour les sels d'autres métaux, par exemple, ceux dérivés du thallium (III) (R. C. Cambie, G. R. Clark, P. A. Craw, P. S. Rutledge, P. D. Woodgate, Aust. J. Chem., 1775 (1984)), on peut estimer que les réactifs générés in situ sont bien les trifluoroacétates, ces réactifs devant se former au fur et à mesure de l'avancement de la réaction. Le milieu réactionnel garde généralement un aspect diphasique. Ensuite l'adjonction du solvant, suivie de l'addition simultanée d'éthérate de trifluorure de bore et du précurseur à cycliser, donne au milieu réactionnel une couleur franche allant du jaune orangé au vert qui atteste de la présence d'ions-radicaux. Des manipulations témoins effectuées sans l'oxyde métallique ont laissé le composé de départ inchangé.

L'exemple 7, détaillé ci-après, décrit la génération in situ du tétrakis(trifluoroacétate) de ruthénium (IV). Typiquement la réaction a lieu dans des solvants pratiquement anhydre tels que l'acétonitrile ou des solvants hydrocarbonés chlorés, par exemple le tétrachlorométhane et le dichlorométhane, selon la solubilité des composés de départ, toutefois c'est ce dernier solvant qui a présenté les meilleurs résultats. Les réactions ont lieu de préférence en atmosphère inerte, sous agitation, à température du laboratoire

i.e. entre 0° et 25 °C. Dans ces conditions, la cinétique est lente (6 h à 24 h). Contrairement à ce qui a été décrit pour le thallium, les mélanges réactionnels obtenus en fin de manipulation sont toujours très propres ; une simple filtration permet de récupérer le catalyseur dégradé qui restait en suspension. Un lavage standard de la phase organique fournit des solutions jaunes pâles, lesquelles, sans chromatographie, permettent d'obtenir des produits bruts très purs. La néoisostégane 2 et le stéganolide A 1 ont ainsi cristallisé spontanément, à l'évaporation du mélange réactionnel brut.

Ainsi, l'utilisation des dérivés du ruthénium (IV), dans les réactions de couplage oxydants biaryliques étudiées, a permis d'obtenir, des rendements supérieurs aux meilleurs réactifs connus (oxyfluorure de vanadium et tris(trifluoroacétate de thallium). Lorsque la réaction de couplage est conduite sous ultrasons, la quantité de catalyseur utilisée est moindre et/ou la durée de réaction est raccourcie.

Il faut ajouter que l'inocuité des sels de ruthénium, permet contrairement aux sels de thallium, une manipulation sans précaution particulière et une récupération facile. Les sels obtenus peuvent ainsi être réoxydés par le métapériodate de sodium pour générer $RuO_4$, réactif très utile en synthèse organique (lui-même recyclable). De plus ces réactions de couplage ont lieu à température ambiante, sans apport énergétique. L'équipement technologique à mettre en œuvre, dans le cas d'une manipulation à grande échelle, n'est pas sophistiqué.

Remarques sur le ruthénium (IV) et ses dérivés :

Il existe deux variétés commerciales de dioxyde de ruthénium. La forme hydratée soluble donne des résultats nettement supérieurs à la forme insoluble, laquelle reste cependant utilisable dans ces réactions de couplage, mais avec des temps de réaction prolongés.

On précisera également que, selon le degré d'hydratation de $RuO_2$, on utilisera le catalyseur selon l'invention à raison de 1 à 10 équivalents-molaires de $RuO_2$ par mole de précurseur biarylique à cycliser.

De manière à mieux comprendre le procédé de synthèse selon l'invention, on indiquera ci-après le schéma réactionnel général dans le cas de la préparation de lignanes bisbenzocyclooctadiènes, suivi de quelques exemples particuliers de mise en œuvre du procédé de couplage selon l'invention.

(Voir Schéma page 10)

## Exemple 1

Préparation de l'hydroxyméthyl-1 triméthoxy-2,3,4 benzène

Dans un tricol de 100 ml équipé d'une agitation magnétique, d'une entrée de gaz et d'un thermomètre, on place 2 g de triméthoxy-2,3,4 benzaldéhyde commercial que l'on dissout dans 15 ml de $CH_2Cl_2$ + 5 ml d'éthanol. On ajoute à température ambiante 200 mg de $NaBH_4$ (6 mmoles) par petites portions. On agite pendant 30 minutes à température ambiante. La CCM (cyclohexane/AcOEt 6/4) indique un produit monotache. On évapore l'éthanole et on reprend par $CHCl_3$, les phases organiques sont lavées avec NaCl puis $H_2O$, séchées sur $MgSO_4$ et évaporées. On obtient une huile jaune pâle analytiquement pure (R = 98 %).

## Exemple 2

Préparation du bromure de triméthoxy-2,3,4 benzyle

Dans un tricol muni d'un septum et équipé d'une agitation magnétique, on injecte sous balayage d'argon une solution éthérée anhydre (10 ml) d'hydroxyméthyl-1 triméthoxy-2,3,4 benzène (1 g, 5 mmoles). On ajoute alors du tribromure de phosphore à — 20 °C (1,46 g, 5 mmoles) en solution dans l'éther (8 ml). Le mélange est agité à — 10 °C pendant 2 heures, puis lavé avec de l'eau glacée et enfin avec NaCl, puis séché sur $MgSO_4$. L'évaporation à O °C, sous pression réduite, donne une huile incolore (1,24 g) homogène en CCM (R = 95 %).

## Exemple 3

Préparation du (triméthoxy-2,3,4 benzyl)-2 (diméthoxy-3,4 benzyl-3 butanolide-4

Dans un tricol séché à la flamme et équipé d'une agitation magnétique, d'une entrée de gaz et d'un septum, on introduit du THF fraîchement distillé sur Na (3 ml), 1,6 ml de Buli 1,6 M (2,54 mmoles), on refroidit à — 80 °C et additionne goutte à goutte de la diisopropylamine fraîchement distillée (0,41 ml). On remonte la température à — 20 °C pendant 20 minutes, on refroidit ensuite à — 80 °C et additionne très lentement, goutte à goutte une solution dans le THF (4 ml) de la vératryl lactone (500 mg, 2,11 mmoles). L'agitation est maintenue pendant 1 heure à — 80 °C, puis l'on remonte la température à — 40 °C pendant 15 minutes et refroidit à nouveau à — 80 °C. On ajoute alors très lentement, à — 80 °C, 0,551 g de bromure de triméthoxy-2,3,4 benzyle (2,11 mmoles) en solution dans du tétrahydrofurane

Schéma réactionnel de préparation de lignanes bisbenzocyclooctadiènes lactoniques

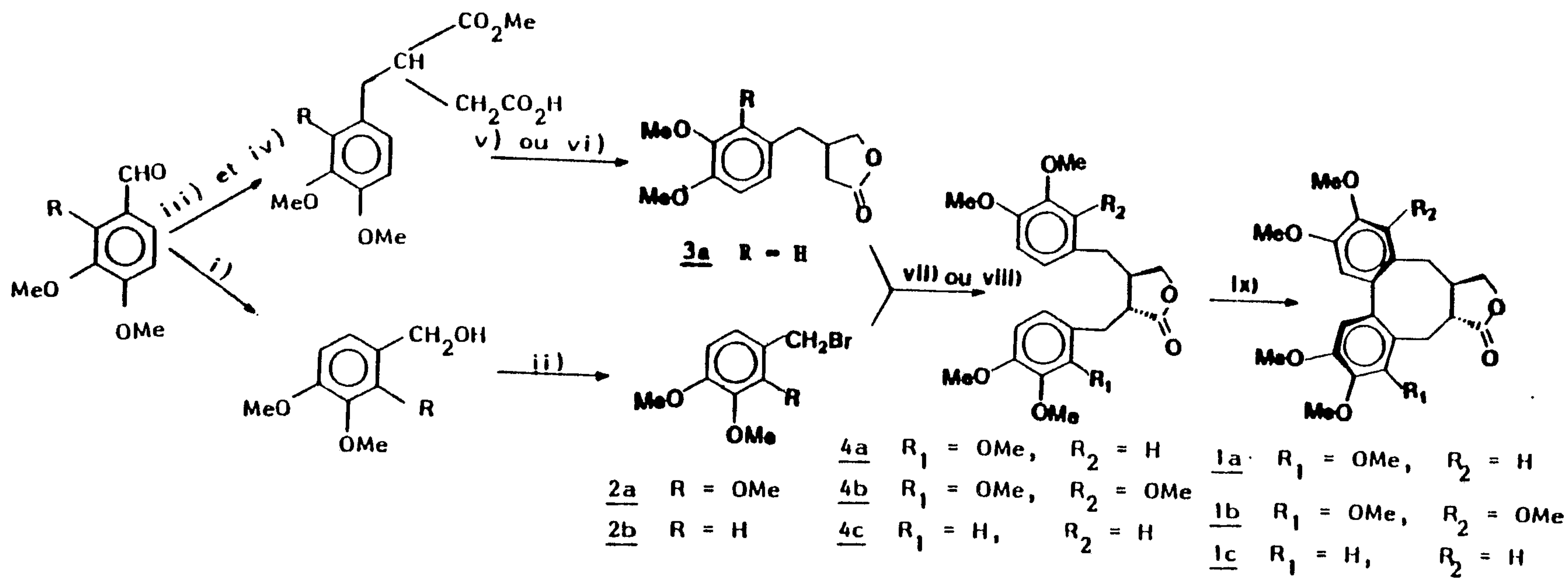


3a R = H

2a R = OMe

2b R = H

4a $R_1$ = OMe, $R_2$ = H

4b $R_1$ = OMe, $R_2$ = OMe

4c $R_1$ = H, $R_2$ = H

1a $R_1$ = OMe, $R_2$ = H

1b $R_1$ = OMe, $R_2$ = OMe

1c $R_1$ = H, $R_2$ = H

Conditions expérimentales

i) $NaBH_4$/MeOH—$CH_2$—$Cl_2$, 0 °C, 5 min. ; $NH_4Cl$
ii) $PBr_3$/Ether-Pyridine, reflux, 2 h
iii) $(CH_2CO_2Me)_2$, MeONa/MeOH, — 10 °C, 3 h ; HCl
iv) $H_2$—Pd—C/AcOEt, 20 °C, 6 h
v) KOH, $CaCl_2$—$NaBH_4$/EtOH, 0 °C, 6 h ; HCl 6N, 20 °C, 30 min.
vi) KOH, $LaCl_3$—$NaBH_4$/EtOH, — 40 °C, 5 min. ; HCl 6N, 20 °C, 30 min.
vii) $LiN(iPr)_2$/THF—HMPT, — 80 °C, 3 h ; HCl 6N, — 30 °C
viii) $LiN[Si(CH_3)_3]$THF/HMPT, — 80 °C, 30 min. ; HCl 6N, — 30 °C
ix) $RuO_2$, $CF_3CO_2H(CF_3CO)_2O$—$BF_3OEt_2/CH_2Cl_2$, 20 °C, 12 à 48 h.

anhydride (2,5 ml) contenant un équivalent d'hexaméthylphosphoramide (0,37 ml). On laisse la température remonter graduellement pendant la nuit, le mélange est refroidi, neutralisé ($NH_4Cl$), la phase organique est lavée à la saumure, puis séchée ($MgSO_4$). Après évaporation et chromatographie du mélange sur silice (toluène/AcOEt, 94/6), on récupère le dibenzylbutanolide sous forme d'une huile épaisse qui, après trituration dans le mélange dichlorométhane-éther de pétrole, abandonne des cristaux translucides F = 113,5-114,5 °C (R = 74 %).

Exemple 4

Préparation du néoisostégane

Dans un tricol de 100 ml équipé d'une agitation magnétique, sous balayage d'argon, on place 192 mg (1,44 mmole) d'oxyde de ruthénium hydraté (forme soluble) en suspension dans 12 ml de dichlorométhane. On ajoute, à température ambiante 1,5 ml (19 mmoles) d'acide trifluoroacétique et 0,8 ml (5,7 mmoles) d'anhydride trifluoroacétique. A la suspension fortement agitée, on ajoute ensuite, à température ambiante, 100 mg (0,24 mmole) de (triméthoxy-2,3,4 benzyl-2 (diméthoxy-3,4 benzyl)-3 butanolide-4 en solution dans 8 ml de dichlorométhane anhydre, immédiatement l'éthérate de trifluorure de bore (0,2 ml, 1,6 mmole). Le surnageant de la solution prend au bout de quelques minutes une coloration jaune pâle. La suspension est maintenue ainsi agitée, une nuit à température ambiante, temps au bout duquel la surveillance en chromatographie sur couche mince indique un produit unique avec disparition complète du produit de départ. On traite le mélange réactionnel par $NaHCO_3$, puis on filtre sur papier afin d'éliminer les résidus solides. La phase organique est alors décantée, et la phase aqueuse extraite à l'acétate d'éthyle. Les phases organiques sont ensuite réunies, séchées sur $MgSO_4$ et évaporées pour donner un solide jaune pâle, pure en chromatographie. Après filtration sur silice (dichlorométhane), on obtient un solide blanc (m = 98 mg, Rendement : 98 %) en tout point comparable (chromatographie, IR, RMN$^1$H) au composé obtenu à partir de l'espèce végétale Steganotaenia araliacea Hochst. (Apiacées), par chromatographie liquide analytique à haute résolution d'un extrait éthanolique.

Exemple 5

Préparation du triméthoxy-2,3,4 benzylidène hémisuccinate de méthyle

Dans un tricol de 250 ml, équipé d'une agitation magnétique, d'un thermomètre, d'une entrée de gaz et d'un réfrigérant, on place 2,8 g (0,12 mole) de sodium fraîchement coupé, puis on ajoute goutte à goutte 40 ml de méthanol distillé sur magnésium. On maintient l'agitation pendant l'addition de MeOH puis on porte à reflux pour compléter la dissolution du Na. Lorsque celui-ci est totalement dissous, on distille la majeure partie du méthanol, puis on ajoute rapidement un mélange chaud de triméthoxy-2,3,4 benzaldéhyde commercial (15 g, 0,076 mole) et de succinate de diméthyle (16 g, 0,106 mole). La réaction est portée à reflux pendant 5 heures, puis, après refroidissement, on acidifie avec HCl à 10 % (refroidi à — 20 °C). La phase organique est décantée et la phase aqueuse extraite au dichlorométhane. Les phases organiques récupérées sont séchées ($MgSO_4$) et évaporées sous pression réduite. L'huile obtenue, après trituration dans l'éther, laisse déposer des cristaux jaunes pâles F = 104-105 °C (éther).

Exemple 6

Préparation du triméthoxy-2,3,4 benzyl hémisuccinate de méthyle

Dans une bouteille à hydrogéner, on place 20 g (0,08 mole) de triméthoxy -2,3,4 benzylidène hémisuccinate de méthyle brut (exemple 5), en solution dans un mélange AcOH/AcOEt 1/1 (100 ml) et additionné de palladium-charbon 10 % (2 g). La bombe à hydrogéner est placée dans un appareil de Parr ($344,75 \cdot 10^3$ Pa), l'agitation est poursuivie pendant 10 heures à température ambiante. Après double filtration sur papier et élimination du solvant sous pression réduite, on récupère l'hémiester saturé brut sous forme d'un solide blanc (Rendement : 97 %).

Exemple 7

Préparation de triméthoxy-2,3,4 benzyl-2 butanolide-4

Dans un tricol équipé d'une agitation magnétique, d'une entrée de gaz, d'un thermomètre et d'une ampoule à addition, on dissout 13,0 g (42 mmoles) de triméthoxy-2,3,4 benzyl hémisuccinate de méthyle (exemple 6) dans 400 ml d'éthanol absolu. On ajoute 2,35 g de KOH en pastille (42 mmoles) et après dissolution totale, on ajoute (10,3 g, 42 mmoles) de chlorure de lanthane pulvérulent ($LaCl_3$). Le mélange fortement agité est refroidi à — 40 °C, puis on ajoute alors goutte à goutte, avec précaution, du borohydrure de sodium (1,4 g, 32 mmoles) en solution dans l'éthanol refroidi à 0 °C (l'addition est accompagnée d'un violent dégagement gazeux). Dès l'addition terminée, on acidifie avec HCl 50 % à froid

et on agite le mélange pendant 1 heure à 20 °C. L'éthanol est alors évaporé et la phase aqueuse extraite au dichlorométhane. Les phases organiques sont lavées, séchées puis évaporées. On récupère une huile mobile, jaune pâle (9,83 g, Rendement = 88 %) qui, triturée dans l'éther, fournit un gâteau cristallin F = 59,5-60,5 (éther) IR (nujol) 1778 (CO lactone) ; 1601 (C = C) ; 1263 RMN ($CDCl_3$) ; 2,07 à 3,07 ppm (m, 5H) aliphatique ; 3,81 ppm (s, 3H) $CH_3O$ ; 3,87 à 4,5 ppm (m, 2H) H aliphatique ; 6,73 à 7,11 ppm (m, 3H) H aromatique ; 7,18 à 7,5 ppm (m, 1H) H aromatique.

Exemple 8

Préparation de bis(triméthoxy-2,3,4 benzyl)-2,3 butanolide-4

Dans un tricol séché à la flamme et équipé d'une agitation magnétique, d'une entrée de gaz et d'un septum, on introduit du THF fraîchement distillé sur Na (30 ml), 16 ml de Buli 1,6 M (25,4 mmoles), puis on refroidit à — 80 °C et on additionne rapidement 1 équivalent d'hexaméthyldisilazane sec. On remonte alors la température à — 40 °C pendant 1 minute, puis on refroidit à — 80 °C et on additionne goutte à goutte, le triméthoxy-2,3,4 benzyl-2 butanolide-4 (5,85 g, 22 mmoles), dans 20 ml de THF anhydre (dibenzyl butanolide issu de l'exemple 7). L'agitation est maintenue pendant 1 heure à — 80 °C, puis l'on remonte la température à —40 °C pendant 15 minutes et on refroidit à nouveau à — 80 °C. On ajoute 5,74 g (22 mmoles) de bromure de triméthoxy-2,3,4 benzyle (exemple 2) en solution dans du tétrahydrofurane anhydre (15 ml) contenant un équivalent d'hexaméthylphosphoramide (3,5 ml). On laisse la température remonter graduellement jusqu'à — 30 °C, puis on neutralise par HCl dilué. La phase organique est lavée à la saumure, puis séchée sur $MgSO_4$. Après évaporation et chromatographie du mélange sur silice (toluène-AcOEt), on récupère 8,33 g de dibenzylbutanolide, sous forme d'un verre incolore homogène en CCM (Rendement = 91 %), qui dissous dans l'éther, laisse déposer de fins cristaux blancs F = 87-88 °C IR (nujol, 1772, 1 601 $cm^{-1}$, RMN$^1$H ($CDCl_3$) 2,2 à 3,5 (6H, m) H aliphatique ; 3,81 (3H, s) OMe ; 3,85 (6H, s) OMe × 2 ; 3,90 (3H, s) OMe ; 3,92 (3H, s) OMe ; 3,94 (3H, s) OMe ; 4,0 à 4,5 (2H, m) —$CH_2$OCO— ; 6,53 à 7,11 (4H, m) H aromatique.

Exemple 9

Préparation du Stéganolide A

Dans un tricol équipé d'une agitation magnétique, sous balayage d'argon, on place 72 mg (0,54 mmole) de $RuO_2$ hydraté (forme soluble) en suspension dans 12 ml de dichlorométhane. On ajoute à température ambiante 0,7 ml (9 mmoles) d'acide trifluoroacétique (TFA) et 0,4 ml d'anhydre trifluoroacétique. A la suspension, on ajoute ensuite, à température ambiante, 40 mg (0,09 mmole) de bis(triméthoxy-2,3,4 benzyl)-2,3 butanolide-4 en solution dans 8 ml de dichlorométhane anhydre et, extemporanément, l'éthérate de trifluorure de bore (0,1 ml, 0,8 mmole). Après une nuit à température ambiante, la surveillance chromatographique sur couche mince indique un produit unique. On traite le mélange réactionnel comme dans l'exemple 4. L'huile jaune obtenue après concentration sous pression réduite et dissolution à chaud dans le mélange dichlorométhane-éther abandonne par refroidissement le stéganolide A racémique sous forme de délicats prismes translucides F = 173-175 °C (Rendement = 94 %). Ce stéganolide A synthétique s'est avéré en tout point comparable au composé d'origine naturelle (voir ci-après exemple 11).

MS $M^+$ = 444,1791 ($C_{24}H_{28}O_8$) ; IR ($CHCl_3$) 1 776 $cm^{-1}$ RMN$^1$H 500 MHz ($CDCl_3$) (δppm) : 6,52 (2H, s) H-1 et H-12 ; 4,39 (1H, dd, $J_{13a-13b}$ = 8,3 Hz, $J_{13a-6}$ = 6,7 Hz) H-13a ; 3,95 (3H, s) ; 3,93 (3h, s) ; 3,92 (3H, s) et 3,86 (6H, s) OMe-2, OMe-3, OMe-4, OMe-9, OMe-10 et OMe-11 ; 3,79 (1H, dd, $J_{13a-13b}$ = 8,3 Hz ; $J_{13b-6}$ = 11,0 Hz) H-13b ; 3,66 (1H, d, $J_{8b-8a}$ = 13,2 Hz) H-8b ; 3,14 (1H, d, $J_{5a-5b}$ = 12,9 Hz) H-5a ; 2,08 (1H, m) H-6 ; 2,01 (1H, dd, $J_{7-8a}$ = 9,0 Hz, $J_{7-6}$ = 13,4 Hz) H-7 ; 1,97 (1H, dd, $J_{5b-6}$ = 9,5 Hz, $J_{5a-5b}$ = 12,9 Hz) H-5b ; 1,91 (1H, dd, $J_{8a-8b}$ = 13,2 Hz, $J_{8a-7}$ = 9,0 Hz) H-8a.

Exemple 10

Atropoisomérisation thermique du stéganolide A

Dans un tube clos équipé d'une vanne, on effectue un balayage d'argon pendant 10 minutes, et on place 20 mg d'un mélange de stéganolide A. Le composé, gardé sous légère surpression d'argon, est alors chauffé dans un bain métallique pendant 2 heures à 220 °C. Après refroidissement l'huile brune obtenue est reprise directement par $CDCl_3$ en vue de l'analyse en RMN 90 MHz. L'étalement du spectre sur 5 ppm dans la région des aromatiques et l'agrandissement des pics permet de mesurer par intégration le rapport des deux atropoisomères iso/normal (85/15).

Exemple 11

Isolement du Stéganolide A à partir de Steganotaenia araliacea

L'étude approfondie en chromatographie liquide analytique à haute résolution (Li-chrocart, Lichrospher CH-8/2 Merck, 5 μ, 250 × 4 mm, 1,2 ml/min., 28 · $10^6$ Pa, gradient ternaire $H_2O$—MeOH—$CH_3CN$) d'une fraction active de l'extrait éthanolique d'un échantillon d'origine ouest-africaine de Steganotaenia araliacea (Apiacées) a permis de déceler de nouveaux composés minoritaires.

L'isolement du stéganolide A sera décrit ci-après.

La chromatographie préparative à haute performance (Hibar RP8 Merck 7 μ, 250 × 25 mm, MeOH—$H_2O$, 12,5 ml/min.), a permis d'obtenir un composé amorphe, MS $M^+$ = 444,1791 ($C_{24}H_{28}O_8$) ; $[\alpha]_D^{20}$ + 67,9° (C = 1,65 ; $CHCl_3$) ; IR ($CHCl_3$) 1 776 $cm^{-1}$ RMN¹H 500 MHz ($CDCl_3$) (δppm) : 6,52 (2H, s) H-1 et H-12 ; 4,39 (1H, dd, $J_{13\alpha-13\beta}$ = 8,3 Hz, $J_{13\alpha-6}$ = 6,7 Hz) H-13α ; 3,95 (3H, s) ; 3,93 (3H, s) ; 3,92 (3H, s) et 3,86 (6H, s) $OCH_3$-2, $OCH_3$-3, $OCH_3$-4, $OCH_3$-9, $OCH_3$-10 et $OCH_3$-11 ; 3,79 (1H, dd, $J_{13\alpha-13\beta}$ = 8,3 Hz ; $J_{13\alpha-6}$ = 11,0 Hz) H-13β ; 3,66 (1H, d, $J_{8\beta-8\alpha}$ = 13,2 Hz) H-8β ; 3,14 (1H, d, $J_{5\alpha-5\beta}$ = 12,9 Hz) H-5α ; 2,08 (1H, m) H-6 ; 2,01 (1H, dd, $J_{7-8\alpha}$ = 9,0 Hz, $J_{7-6}$ = 13,4 Hz) H-7 ; 1,97 (1H, dd, $J_{5\beta-6}$ = 9,5 Hz, $J_{5\alpha-5\beta}$ = 12,9 Hz) H-5β ; 1,91 (1H, dd, $J_{8\alpha-8\beta}$ = 13,2 Hz, $J_{8\alpha-7}$ = 9,0 Hz) H-8α.

L'étude du spectre précédent montre qu'il s'agit d'un biaryle ponté de type bis-benzocyclooctadiéno-lactonique, porteur de 6 méthoxyles aromatiques et comportant un —$CH_2$—en d'un hétéroatome (méthylène lactonique à 3,79 et 4,39 ppm). L'absence de méthoxyle déplacé vers les hauts champs démontre les positions 2, 3, 4, 9, 10, 11 pour ces derniers. L'ensemble du squelette aliphatique a pu être déterminé sans ambiguïté à l'aide d'expériences de double irradiation. Sur le plan stéréochimique, la valeur de 13,4 Hz, pour le couplage $H_6$-$H_7$, montre la présence d'une jonction lactonique trans. L'observation du couplage $H_{8\beta}$—$H_7$ (J = 0 Hz), confirme l'atropisomérie iso tandis que l'absence de couplage benzylique $H_8$-$H_9$ est un argument supplémentaire en faveur de la présence d'un méthoxyle en position 9. Comme pour le néoisostégane, la compression stérique entre l'hydrogène $H_{8\beta}$ et ce méthoxyle, conduit à un éclatement des figures des protons benzyliques $H_{8\alpha}$ et $H_{8\beta}$, se traduisant par une différence de déplacement chimique de 1,75 ppm (contre 1,76 pour ce dernier). Dans le cas du stéganolide A, on observe un phénomène comparable pour l'autre paire de protons benzyliques $H_{5\alpha}$ et $H_{5\beta}$, soit une différence de 1,17 ppm contre 0,26 ppm pour le néoisostégane, en accord avec la situation du méthoxyle sur C-4. Ces éléments spectroscopiques, comparés à ceux déjà obtenus avec les diastéréoisomères des stéganes et le néoisostégane, suggèrent que ce composé est le (+)-(M, 6R, 7R)-stéganolide A.

Exemple 12

Préparation de la glaucine 5 à partir de la laudanosine

Dans un tricol de 100 ml équipé d'une agitation magnétique, d'une entrée de gaz, d'un thermomètre et d'un septum, on introduit 150 mg (1,12 mmole) d'oxyde de ruthénium hydraté en suspension dans 18 ml de $CH_2Cl_2$ anhydre, on ajoute 1,2 ml (16 mmole) d'acide trifluoroacétique et 0,7 ml (4,96 mmole) d'anhydride trifluoroacétique. La suspension est refroidie à —10 °C dans un bain glace-acétone. On ajoute alors 0,1 g (0,28 mmole) de DL Laudanosine en solution dans 6 ml de $CH_2Cl_2$ anhydre, puis immédiatement 0,16 ml (1,3 mmole) de $BF_3$ éthérate. La solution rouge ainsi obtenue est agitée à température ambiante pendant 24 heures. La CCM ($CH_2Cl_2$MeOH 9/1) indiquant la disparition totale du produit de départ. On traite le mélange avec $NH_4OH$ 10 % jusqu'à atteindre pH 9, puis on filtre sur papier la solution hétérogène. La phase organique est décantée et la phase aqueuse extraite avec AcOEt. Les phases organiques sont lavées avec NaCl saturé, $H_2O$, puis séchées sur $MgSO_4$. Après évaporation, on récupère la glaucine brute sous forme d'une huile brune homogène en CCM (Rendement = 92 %) qui après chromatographie sur gel de silice ($CH_2Cl_2$/MeOH 99/1) donne une huile jaune pâle qui cristallise dans un mélange $CH_2Cl_2$/éther (aiguilles) (F = 134-136 °C) (litt. 137-139 °C).

Exemple 13

Préparation de l'analogue 1d de formule

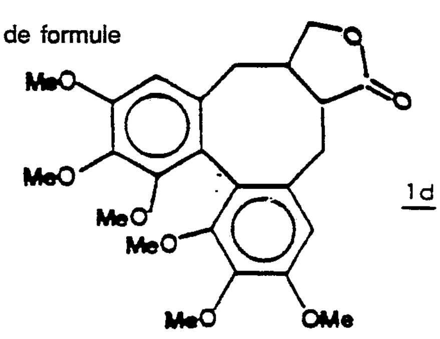

1d

Dans un tricol de 100 ml équipé d'une agitation magnétique, d'une entrée de gaz, d'un septum et d'un thermomètre, on place 0,060 g (0,448 mmole) d'oxyde de ruthénium hydraté en suspension dans 10 ml de $CH_2Cl_2$ anhydre, on ajoute 0,48 ml (6,19 mmoles) d'acide trifluoroacétique et 0,25 ml (1,78 mmole) d'anhydride trifluoroacétique. La suspension est refroidie à — 10 °C à l'aide d'un bain glace-acétone, on ajoute alors 0,1 g (0,224 mmole) de (triméthoxy-3',4',5' benzyl)-3 (triméthoxy-3,4,5 benzyl)-3 butanolide-4 en solution dans 5 ml de $CH_2Cl_2$ anhydre, puis immédiatement 0,0095 ml (0,083 mmole) de $BF_3$ éthérate. Le mélange est agité vigoureusement pendant 24 heures à température ambiante. La CCM (toluène/AcOEt 7/3) montrant la disparition totale du produit de départ, on refroidit la suspension et on traite avec $NaHCO_3$ 5 %, le mélange est filtré et la phase aqueuse extraite avec AcOEt. Les phases organiques réunies sont lavées avec NaCl saturé, $H_2O$ puis séchées sur $MgSO_4$ et enfin évaporées. On obtient ainsi une huile jaune-pâle homogène en CCM (m = 93 mg) (Rendement = 93 %).

Exemple 14

Préparation de l'analogue 1e de formule

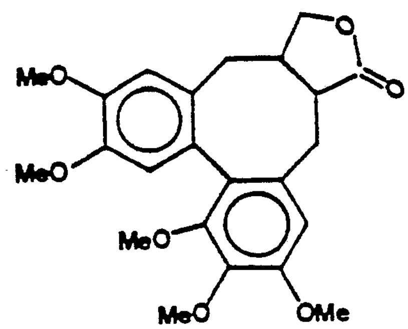

1e

Dans un tricol de 100 ml équipé d'une agitation magnétique, d'une entrée de gaz et d'un thermomètre, on place 0,064 g (0,480 mmole) d'oxyde de ruthénium hydraté en suspension dans 10 ml de $CH_2Cl_2$ anhydre, on ajoute 0,51 ml (6,62 mmole) d'acide trifluoroacétique et 0,27 ml (1,91 mmole) d'anhydride trifluoroacétique. La suspension est ainsi refroidie à — 10 °C à l'aide d'un bain glace-acétone. On ajoute alors 0,1 g (0,24 mmole) de (triméthoxy-3',4',5' benzyl)-3 (diméthoxy-3,4 benzyl)-3 butanolide-4 en solution dans 5 ml de $CH_2Cl_2$ anhydre, puis immédiatement 0,01 ml (0,089 mmole) de $BF_3$ éthérate. Le mélange est agité vigoureusement pendant 24 heures à température ambiante. La CCM (toluène/AcOEt 7/3) montrant la disparition totale du produit de départ, le mélange est refroidi à — 10 °C et traité avec $NaHCO_3$ 5 %, puis filtré sur papier. La phase aqueuse est extraite avec AcOEt et les phases organiques réunies sont lavées avec NaCl saturé, $H_2O$, puis séchées sur $MgSO_4$. Après évaporation du solvant, on obtient une huile jaune-pâle qui cristallise dans un mélange $CH_2Cl_2$/éther abandonnant 94 mg (Rendement = 94 %) d'un solide se présentant sous forme d'aiguilles (F = 213-215 °C).

Etude comparative de différents réactifs de couplage biarylique oxydant

Afin de mettre en évidence les améliorations obtenues par suite de l'utilisation du nouveau catalyseur objet de la présente invention, on indiquera dans le tableau ci-après les résultats comparatifs obtenus avec d'autres catalyseurs, dans le cadre de la synthèse de quelques dérivés particuliers ; pour les analogues 1a, 1b et 1c, le schéma de synthèse se résume comme suit :

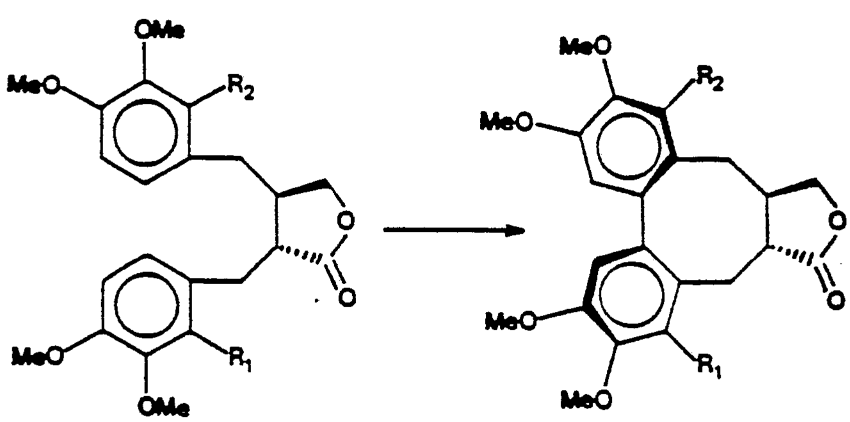

4a $R_1$ = OMe, $R_2$ = H
4b $R_1$ = OMe, $R_2$ = OMe
4c $R_1$ = H, $R_2$ = H
1a $R_1$ = OMe, $R_2$ = H
1b $R_1$ = OMe, $R_2$ = OMe
1c $R_1$ = H, $R_2$ = H

Les synthèses des trois dérivés benzocyclooctadiénolactoniques 1a, 1b et 1c énoncés précédemment ainsi que des analogues de formules 1d et 1e (exemples 13 et 14), et de la glaucine ont été effectuées avec des catalyseurs de couplage différents, dans chaque cas à partir du même précurseur dibenzylbutanolide.

Ces catalyseurs sont respectivement :

l'oxychlorure de vanadium $VOCl_3$ ;
l'oxyfluorure de vanadium $VOF_3$ ;
le tris(trifluoroacétate) de thallium (III) TTFA, et
le tétrakis(trifluoroacétate) de ruthénium (IV) RUTFA.

Tableau de résultats comparatifs

| Composé | Catalyseur | Milieux réactionnels | t(°C) | Temps (h) | R(%) |
|---|---|---|---|---|---|
| Néoisostégane 1a | $VOCl_3$ | $CF_3CO_2H-(CF_3CO)_2O-CH_2Cl_2$ | - 80 | 3 | 50[a] |
| " | $VOF_3$ | $CF_3CO_2H-(CF_3CO)_2O-CH_2Cl_2$ | - 50 | 3 | 46[a] |
| " | TTFA | $CF_3CO_2H-(CF_3CO)_2O-BF_3Et_2O-CH_2Cl_2$ | - 10 | 2 | 75 |
| " | RUTFA[b] | $CF_3CO_2H-(CF_3CO)_2O-BF_3Et_2O-CH_2Cl_2$ | + 20 | 12 | 98 |
| Stéganolide A 1b | TTFA | $CF_3CO_2H-(CF_3CO)_2O-BF_3Et_2O-CH_2Cl_2$ | - 10 | 1 | 69 |
| " | RUTFA | $CF_3CO_2H-(CF_3CO)_2O-BF_3Et_2O-CH_2Cl_2$ | + 20 | 48[c] | 96 |
| Analogue 1c | TTFA | $CF_3CO_2H-(CF_3CO)_2O-BF_3Et_2O-CH_2Cl_2$ | - 10 | 1 | 79[d] |
| " | RUTFA | $CF_3CO_2H-(CF_3CO)_2O-BF_3Et_2O-CH_2Cl_2$ | + 20 | 48 | 97 |
| Analogue 1d | TTFA | $CF_3CO_2H-(CF_3CO)_2O-BF_3Et_2O-CH_2Cl_2$ | - 10 | 1 | 70 |
| " | RUTFA | $CF_3CO_2H-(CF_3CO)_2O-BF_3Et_2O-CH_2Cl_2$ | + 20 | 24 | 93[d] |
| Analogue 1e | TTFA | $CF_3CO_2H-(CF_3CO)_2O-BF_3Et_2O-CH_2Cl_2$ | 0 | 3 | 72[d] |
| " | RUTFA | $CF_3CO_2H-(CF_3CO)_2O-BF_3Et_2O-CH_2Cl_2$ | + 20 | 24 | 94[d] |
| Glaucine 2b | TTFA | $CF_3CO_2H-(CF_3CO)_2O-BF_3Et_2O-CH_2Cl_2$ | 0 | 3 | 65[d] |
| " | RUTFA | $CF_3CO_2H-(CF_3CO)_2O-BF_3Et_2O-CH_2Cl_2$ | + 20 | 24 | 92[d] |

[a] Présence de produits secondaires de dégradation
[b] Non isolé, engendré in situ à partir de $RuO_2 \cdot xH_2O$ (1,5 equiv.)
[c] Temps de réaction environ doublés avec la forme anhydre de $RuO_2$
[d] Déjà décrite dans la technique antérieure (R. C. Cambie, G. R. Clark, P. A. Craw, P. S. Rutledge et P. D. Woodgate, Aust. J. Chem., 37, 1775 (1984).

Les résultats récapitulés dans le tableau ci-dessus démontrent la très nette supériorité du catalyseur selon l'invention (RUTFA) sur les autres agents de couplage connus. Contrairement à ce qui a été décrit à propos des réactifs connus, dans le cas du RUTFA, les mélanges réactionnels obtenus en fin de manipulations sont incolores et l'excès de catalyseur peut être récupéré par simple filtration.

## Revendications

1. Procédé de couplage biarylique oxydant intramoléculaire de composés précurseurs comportant deux noyaux aromatiques, éventuellement polycondensés et pouvant contenir un ou plusieurs hétéroatomes tels que l'oxygène et/ou l'azote, reliés entre eux par une chaîne hydrocarbonée linéaire ou ramifiée, saturée ou insaturée et renfermant éventuellement un ou plusieurs hétéroatomes tels que l'oxygène et/ou l'azote, caractérisé en ce que les précurseurs biaryliques sont cyclisés en présence de tétrakis(trifluoroacétate) de ruthénium (IV).

2. Procédé selon la revendication 1, caractérisé en ce que le tétrakis(trifluoroacétate) de ruthénium

(IV) est généré in situ par réaction de l'acide trifluoroacétique sur le dioxyde de ruthénium $RuO_2$ en présence d'anhydride trifluoroacétique.

3. Procédé selon la revendication 2, caractérisé en ce que la réaction de l'acide trifluoroacétique sur le dioxyde de ruthénium est conduite en outre en présence d'un réactif d'assistance électrophile, en particulier en présence d'éthérate de trifluorure de bore $BF_3Et_2O$.

4. Procédé selon l'une des revendication 1 à 3, caractérisé en ce que l'on utilise le tétrakis(trifluoroacétate) de ruthénium (IV) à raison de 1 à 10 équivalent(s) molaire(s) de dioxyde de ruthénium $RuO_2$ par mole de précurseur biarylique à cycliser.

5. Procédé selon l'une des revendications 1 à 4, caractérisé en ce que la réaction de cyclisation est conduite dans un solvent organique pratiquement anhydre, en particulier un solvant hydrocarboné chloré tel que le dichlorométhane.

6. Procédé selon l'une des revendications 1 à 5, caractérisé en ce que la réaction de cyclisation est conduite sous atmosphère de gaz inerte, en particulier sous argon.

7. Procédé selon l'une des revendications 1 à 6, caractérisé en ce que la réaction de cyclisation est conduite à une température sensiblement comprise entre 0 °C et 25 °C.

8. Procédé selon l'une des revendications 1 à 7, caractérisé en ce que le précurseur biarylique à cycliser est choisi parmi les précurseurs ouverts de lignanes bisbenzocyclooctadiènes, de lignanes bisbenzocyclooctadiènes lactoniques, de tétrahydrophénantrènes, d'alcaloïdes aporphiniques, d'alcaloïdes homoaporphiniques, d'alcaloïdes du groupe de la colchicine, d'alcaloïdes du groupe des dibenzazonines, d'alcaloïdes du groupe des dibenzazécines, d'alcaloïdes du groupe des phénanthroquinolizidines et des alcaloïdes du groupe des phénanthroindolizidines.

9. Procédé selon l'une des revendications 1 à 8, caractérisé en ce que la réaction de cyclisation est conduite sous ultrasons.

10. Procédé selon l'une des revendications 1 à 8, caractérisé en ce que le précurseur biarylique à cycliser est le (triméthoxy-2,3,4 benzyl)-2 (diméthoxy-3,4 benzyl)-3 butanolide-4.

11. Procédé selon l'une des revendications 1 à 8, caractérisé en ce que le précurseur biarylique à cycliser est le bis(triméthoxy-2,3,4 benzyl)-2,3 butanolide-4.

12. A titre de catalyseur nécessaire à la mise en œuvre du procédé selon l'une des revendications 1 à 11, le tétrakis(trifluoroacétate) de ruthénium (IV).

13. Le composé de formule

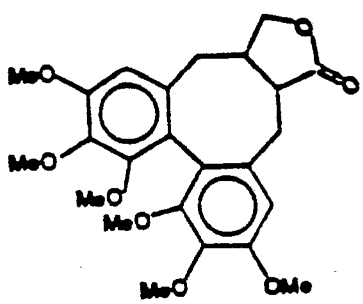

14. Le Stéganolide A de formule

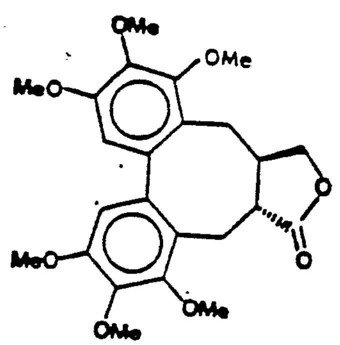

## Claims

1. A method of intramolecular oxidizing biarylic coupling of precursor compounds comprising two aromatic rings, polycondensed if required and if required containing one or more heteroatoms such as oxygen and/or nitrogen interconnected by a straight or branched, saturated or unsaturated hydrocarbon

chain containing one or more heteroatoms such as oxygen and/or nitrogen if required, characterised in that the biarylic precursors are cyclized in the presence of ruthemium (IV) tetrakis (trifluoroacetate).

2. A method according to claim 1, characterised in that the ruthenium (IV) tetrakis trifluoroacetate is generated in situ by reaction of trifluoroacetic acid with ruthenium dioxide $RuO_2$ in the presence of trifluoroacetic anhydride.

3. A method according to claim 2, characterised in that the reaction between trifluoroacetic acid and ruthenium dioxide is also brought about in the presence of an assisting electrophilic reagent, more particularly in the presence of boron trifluoride etherate $BF_3Et_2O$.

4. A method according to any of claims 1 to 3, characterised in that ruthenium (IV) tetrakis (trifluoroacetate) is used in the proportion of 1 to 10 molar equivalents of ruthenium dioxide $RuO_2$ per mol of biarylic precursor to be cyclized.

5. A method according to any of claims 1 to 4, characterised in that the cyclization reaction is brought about in a substantially anhydrous organic solvent, more particularly a chlorinated hydrocarbon solvent such as dichloromethane.

6. A method according to any of claims 1 to 5, characterised in that the cyclization reaction is brought about in an inert gas, more particularly argon, atmosphere.

7. A method according to any of claims 1 to 6, characterised in that the cyclization reaction is brought about at a temperature substantially between 0 °C and 25 °C.

8. A method according to any of claims 1 to 7, characterised in that the biarylic precursor to be cyclized is chosen from among open precursors of bisbenzocyl-cooctadiene lignanes, lactonic bisbenzocyclooctadiene lignanes, tetrahydrophenanthrenes, aporphinic alcaloids, homoaporphinic alcaloids, alcaloids in the colchicine group, alcaloids in the dibenzazonine group, alcaloids in the dibenzazecine group, alcaloids in the phenanthroquinolizidine group and alcaloids in the phenanthroindolizidine group.

9. A method according to any of claims 1 to 8, characterised in that the cyclization reaction is brought about in ultrasound.

10. A method according to any of claims 1 to 8, characterised in that the biarylic precursor to be cyclized is 2-(2,3,4-trimethoxybenzyl) 3-(3,4-dimethoxybenzyl) 4-butanolide.

11. A method according to any of claims 1 to 8, characterised in that the biarylic precursor to be cyclized is bis-2,3(2,3,4 trimethoxybenzyl) 4-butanolide.

12. Ruthenium (IV) tetrakis (trifluoroacetate) as the catalyst necessary for working the method according to any of claims 1 to 11.

13. The compound of the formula

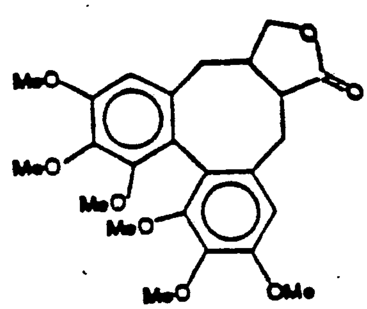

14. The Steganolide A of the formula

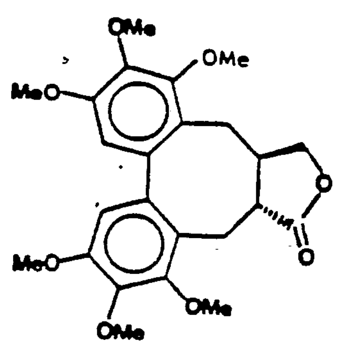

**Patentansprüche**

1. Verfahren zur intramolekularen biarylischen oxidierenden Kupplung von Vorläuferverbindungen, die zwei aromatische Kerne aufweisen, die gegebenenfalls polykondensiert sein können und ein oder mehrere Heteroatome, wie Sauerstoff und/oder Stickstoff enthalten können, die miteinander durch eine

lineare und verzweigte gesättigte oder ungesättigte und gegebenenfalls ein oder mehrere Heteroatome, wie Sauerstoff und/oder Stickstoff einschließende Kohlenwasserstoffkette verbunden sind, dadurch gekennzeichnet, daß die Biaryl-Vorläufer in Gegenwart von Ruthenium (IV)-tetrakis(trifluoracetat) cyclisiert werden.

2. Verfahren nach Anspruch 1, dadurch gekennzeichnet, daß das Ruthenium (IV)-tetrakis(trifluoracetat) in situ erzeugt wird durch Reaktion von Trifluoressigsäure mit Rutheniumdioxid $RuO_2$, in Anwesenheit von Trifluoressigsäureanhydrid.

3. Verfahren nach Anspruch 2, dadurch gekennzeichnet, daß die Reaktion der Trifluoressigsäure mit dem Rutheniumdioxid außerdem in Anwesenheit eines elektrophilen Hilfsreagens, insbesondere in Anwesenheit von Bortrifluorid-Etherat, $BF_3$ $Et_2O$ durchgeführt wird.

4. Verfahren nach einem der Ansprüche 1 bis 3, dadurch gekennzeichnet, daß man das Ruthenium(IV)-tetrakis(trifluoracetat) in einer Menge von 1 bis 10 Moläquivalent(en) von Rutheniumdioxid $RuO_2$, pro Mol des zu cyclisierenden Biaryl-Vorläufers, verwendet.

5. Verfahren nach einem der Ansprüche 1 bis 4, dadurch gekennzeichnet, daß die Cyclisierungsreaktion in einem praktisch wasserfreien organischen Lösungsmittel, insbesondere in einem chlorierten Kohlenwasserstofflösungsmittel, wie Dichlormethan, durchgeführt wird.

6. Verfahren nach einem der Ansprüche 1 bis 5, dadurch gekennzeichnet, daß die Cyclisierungsreaktion unter einer Inertgas-Atmosphäre, insbesondere unter Argon, durchgeführt wird.

7. Verfahren nach einem der Ansprüche 1 bis 6, dadurch gekennzeichnet, daß die Cyclisierungsreaktion bei einer Temperatur zwischen 0 °C und 25 °C durchgeführt wird.

8. Verfahren nach einem der Ansprüche 1 bis 7, dadurch gekennzeichnet, daß der zu cyclisierende Biaryl-Vorläufer ausgewählt wird unter den offenen Vorläufern von Bisbenzocyclooctadien-lignanen, lactonischen Bisbenzocyclooctadien-lignanen, Tetrahydrophenanthrenen, Aporphinalkaloiden, Homoapophin-alkaloiden, Alkaloiden aus der Colchicin-Gruppe, Alkaloiden aus der Gruppe von Dibenzazoninen, Alkaloiden aus der Gruppe von Dibenzazecinen, Alkaloiden aus der Gruppe von Phenanthrochinolizidinen und Alkaloiden aus der Gruppe von Phenanthroindolizidinen.

9. Verfahren nach einem der Ansprüche 1 bis 8, dadurch gekennzeichnet, daß die Cyclisierungsreaktion unter Ultraschall durchgeführt wird.

10. Verfahren nach einem der Ansprüche 1 bis 8, dadurch gekennzeichnet, daß der zu cyclisierende Biaryl-Vorläufer das 2-(2,3,4-Trimethoxybenzyl)-3-(3,4-dimethoxybenzyl)-butanolid-4 ist.

11. Verfahren nach einem der Ansprüche 1 bis 8, dadurch gekennzeichnet, daß der zu cyclisierende Biaryl-Vorläufer das Bis-2,3-(2,3,4-trimethoxybenzyl)-butanolid-4 ist.

12. Ruthenium (IV)-tetrakis(trifluoracetat) als Katalysator, der zur Durchführung des Verfahrens nach einem der Ansprüche 1 bis 11 benötigt wird.

13. Verbindung der Formel

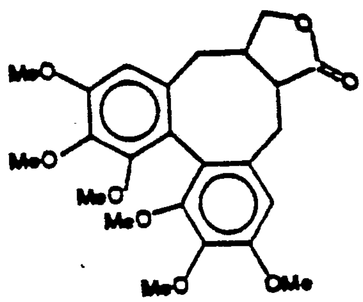

14. Steganolid A der Formel